# FASCICULE DE BREVET EUROPEEN

(11) **EP 1 708 653 B1**
(45) Date de publication et mention de la délivrance du brevet: **30.09.2009**
(21) Numéro de dépôt: 04817623.4
(22) Date de dépôt: 20.12.2004
(51) Int. Cl.: A61F 2/42

(54) **PROTHESE INTERPHALANGIENNE ET/OU METACARPOPHALANGIENNE**
INTERPHALANGEALE UND/ODER METACARPOPHALANGEALE GELENKSPROTHESE
INTERPHALANGEAL AND/OR METACARPOPHALANGEAL PROSTHESIS

(30) Priorité: 22.12.2003 FR 0315635
(43) Date de publication de la demande: 11.10.2006
(73) Titulaire: Memometal Technologies, F-35170 Bruz (FR)
(72) Inventeur: BELLEMERE, Philippe, F-44000 Nantes (FR); PRANDI, Bernard, F-35700 Rennes (FR)
(74) Mandataire: Thivillier, Patrick
(86) Numéro de dépôt international: PCT/FR2004/050735
(87) Numéro de publication internationale: WO 2005/063149

(56) Documents cités:
- US-A- 5 824 095
- US-A- 6 007 580

## Description

### Domaine Technique

L'invention concerne un dispositif chirurgical permettant de remplacer une articulation endommagée ou bien arthrosée entre deux phalanges de la main et/ou du pied entre deux phalanges. Le document US 6 007 580 est considéré comme étant l'état de la technique le plus proche.

Elle vise plus particulièrement une partie déformable située entre les deux parties d'ancrage.

### Art antérieur

De façon générale, les prothèses interphalangiennes se présentent sous la forme de deux portions d'ancrage reliées soit par une liaison mécanique, soit par une zone déformable.

Dans une forme de réalisation connue, la prothèse présente une charnière autorisant le mouvement d'un doigt dans un plan en flexion-extension. Avec ce type de prothèse, l'axe de rotation est fixe alors que celui d'une articulation saine est mobile.

Enfin, un suivi à dix ans de ces prothèses montre des dégradations progressives avec perte de la mobilité, et dans au moins la moitié des cas, rupture de la prothèse, desaxation digitale et résorption osseuse.

Pour tenter de remédier à ces inconvénients, on a proposé des implants en silicone monobloc flexible. Ce concept est destiné à améliorer et à stabiliser la résection arthroplastique : une encapsulation fibreuse s'organise autour de l'implant, permettant le maintien de l'alignement et de l'écart articulaire quand la mobilisation débute.

Un tel type d'implant est décrit dans le document EP 1 300 122 dans lequel la portion centrale flexible est formée d'un matériau silicone. Cependant, après un recul de cinq ans, d'importantes ruptures nécessitent l'interposition de bagues en titane pour limiter l'usure des tiges. Malgré ces différentes améliorations, la durée de vie de l'implant est courte : la mobilité diminue en deux ans, et il est fréquent de constater une rupture de l'implant ou une migration de celui-ci dans la diaphyse.

Enfin, il a également été décrit des prothèses présentant une charnière constituée d'un ressort hélicoïdal situé dans une portion centrale selon une direction transversale ou longitudinale. Ainsi, le document EP 0 959 819 décrit une prothèse comportant des ressorts dans une portion centrale orientée selon l'axe des tiges. La prothèse ainsi conçue permet donc la flexion-extension, le mouvement en intégralité, la compression et la traction de la partie centrale. Cependant, avec une telle prothèse, le problème de la stabilité se pose lorsque le doigt est fléchi et que l'on exerce une compression à l'extrémité de la dernière phalange.

On peut citer également le brevet US 6 007 580 qui divulgue un spacer de polymère biodégradable susceptible de se désagréger dans le corps humain. Compte tenu de la duré de biodégradabilité qui est d'environ un an, on peut considérer qu'il n'y aura plus, entre les deux parties d'ancrage dans l'os, qu'un trou plus ou moins rempli par du tissu mou. Il en résulte un problème de force de stabilité latérale et de flexibilité dorsale.

### Exposé de l'invention

L'invention s'est fixée pour but de remédier à ces inconvénients de manière simple, sûre et efficace.

Le problème que se propose de résoudre l'invention est de former un implant qui présente les mêmes degrés de mobilité qu'une articulation saine, qui soit stable en compression, qui autorise une longévité supérieure et absorbe les chocs, et limite la transmission des contraintes à l'os.

Pour résoudre un tel problème, il a été conçu et mis au point une prothèse interphalangienne et/ou métacarpophalangienne comportant une portion centrale déformable et deux parties d'ancrage situées de part et d'autre de cette portion centrale. La portion centrale comporte au moins deux volets articulés l'un par rapport à l'autre, en faisant office de plis aptes à permettre au moins un mouvement de flexion.

Autrement dit, la partie centrale de la prothèse se présente sous forme sensiblement d'un accordéon et présente au moins deux volets aptes à s'écarter l'un de l'autre, à se rapprocher et à pivoter. Ce pivotement permet en effet de réaliser le mouvement de flexion / extension de l'articulation. Le rapprochement des deux volets permet quant à lui d'absorber les chocs ou d'autoriser une compression de l'articulation qui est stable, et ce même en flexion.

De plus la partie centrale permet un mouvement de latéralité comme une articulation saine. Cette partie permet également un mouvement en compression et traction qui limite la transmission des contraintes à l'os et donc limite le descellement des ancrages.

La prothèse peut être choisie dans un matériau comportant une grande élasticité tel qu'un alliage à mémoire de forme.

Autrement dit, le déplacement des volets l'un par rapport à l'autre peut être effectué à l'aide d'une grande amplitude sans que les contraintes internes ne dépassent le seuil du domaine élastique.

En pratique, l'alliage à mémoire de forme peut comporter du titane et du nickel.

Avantageusement, chaque volet est apte à se déformer en torsion. Ainsi, le mouvement en latéralité au niveau de l'articulation est possible et l'amplitude peut être déterminée en fonction de la forme de l'épaisseur ou de la largeur de chaque volet.

En pratique, afin de pouvoir ajuster le mouvement de latéralité, les volets de la portion centrale peuvent être ajourés longitudinalement. Autrement dit, chaque volet peut être dédoublé de façon à générer un vide sur toute la longueur de la portion centrale entre deux doublons de volets.

En pratique, la section des volets peut être variable. Autrement dit, chaque volet peut comporter une épaisseur variable. A titre d'exemple, il est possible d'utiliser des volets qui présentent une épaisseur plus importante dans la zone médiane située entre deux plis pour lesquels l'épaisseur a pu être réduite pour autoriser une plus grande amplitude de déformation.

Avantageusement, les plis peuvent être symétriques entre eux. Autrement dit, lorsque l'on regarde de profil la portion centrale de la prothèse, la forme des plis situés dans la partie supérieure de la prothèse sont sensiblement identiques à ceux situés dans la partie inférieure de la prothèse.

Selon un autre mode de réalisation, les plis peuvent également être asymétriques entre eux. Autrement dit, et contrairement à l'exemple précédent, les plis situés dans la partie supérieure peuvent présenter une géométrie différente de ceux situés dans la partie inférieure de la prothèse.

Concernant la localisation des plis par rapport aux parties d'ancrage, plusieurs modes de réalisation sont également envisageables.

Dans un premier mode de réalisation, les plis peuvent être disposés symétriquement par rapport à un plan contenant les axes des deux parties d'ancrage.

Selon un autre mode de réalisation, les plis peuvent également être disposés de manière décalée par rapport à un plan contenant les axes des parties d'ancrage. Autrement dit, les zones inférieure et supérieure des plis se situent à des distances différentes d'un plan contenant les axes des deux parties d'ancrage.

Enfin plusieurs configurations et modes de réalisation sont possibles pour cette prothèse. Ainsi elle peut, dans une première variante, se présenter sous la forme d'un ensemble monobloc, les deux parties d'ancrage et la portion centrale étant solidaires.

Dans une seconde variante, il est possible de rapporter une portion centrale entre deux parties d'ancrage de façon à permettre le changement ultérieur de cette portion, par exemple lorsque la mobilité de l'articulation n'est plus adaptée ou plus simplement lorsque la portion centrale a perdu de son élasticité.

### Description sommaire des figures

La manière de réaliser l'invention ainsi que les avantages qui en découlent ressortiront bien de la description du mode de réalisation qui suit, donné à titre indicatif et non limitatif, à l'appui des figures annexées dans lesquelles :
La figure 1 est une vue en perspective d'une prothèse implantée dans un doigt, conformément à l'invention.
La figure 2 représente une vue en perspective schématique de la portion centrale d'une prothèse, conformément à l'invention.
La figure 3 est une vue en perspective de la portion centrale de la prothèse symbolisant le mouvement de flexion, conformément à l'invention.
La figure 4 est une vue en coupe schématique de la portion centrale de la prothèse, symbolisant la capacité de mouvement en latéralité de la prothèse, conformément à l'invention.
Les figures 5 à 7 représentent des vues de profil schématiques de plusieurs variantes d'une prothèse, conforme à l'invention.
La figure 8 est une vue en perspective d'une prothèse comportant une partie centrale ajourée.

### Manière de décrire l'invention

Comme déjà évoqué, l'invention concerne une prothèse interphalangienne ou métacarpophalangienne comportant une portion centrale déformable (1) et deux parties d'ancrage (2) et (3) situées de part et d'autre de cette portion centrale. Avantageusement, la prothèse est réalisée dans un matériau comportant une grande élasticité tel qu'un alliage à mémoire de forme à base de titane et de nickel. L'alliage à mémoire de forme est sélectionné pour que le taux local de la déformation maximum soit très inférieur à la limite de fatigue, selon la courbe WHOLER.

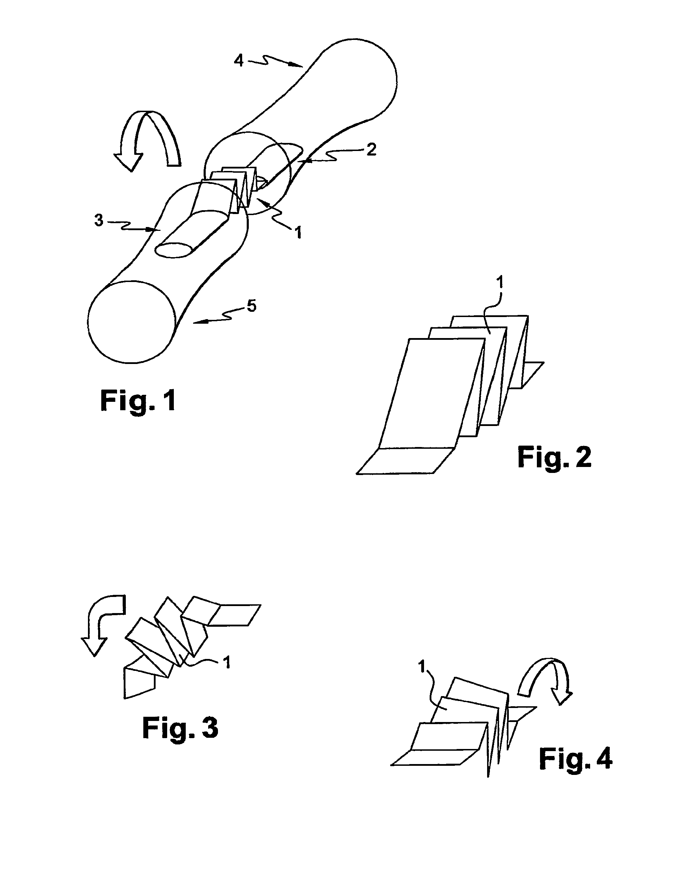

Tel que décrit à la figure 1, la prothèse est implantée entre deux phalanges (4,5), la partie d'ancrage (2) étant insérée dans la phalange (4), tandis que la seconde partie d'ancrage (3) est insérée dans la phalange (5). La portion centrale (1) comporte au moins deux volets articulés l'un par rapport à l'autre, et raccordés par des plis aptes à permettre au moins un mouvement de flexion. Les volets sont alors aptes à se déplacer à la manière d'un accordéon se rapprochant l'un de l'autre pour permettre une compression de l'articulation, et peuvent pivoter l'un par rapport à l'autre pour permettre la flexion / extension.

Tel que représenté schématiquement à la figure 2, les volets peuvent être formés par le pliage d'une bande selon deux directions, dans une certaine limite car des butées peuvent limiter le mouvement (par exemple en extension).

Tel que représenté schématiquement à la figure 3, la portion centrale (1) peut se déformer afin d'autoriser la flexion d'un doigt. Pour ce faire, chaque volet peut pivoter l'un par rapport à l'autre autour des plis qu'elle comporte. Cette géométrie particulière permet en outre de fournir une grande stabilité en compression de l'implant lorsque celui-ci est en position fléchie.

Tel que représenté schématiquement à la figure 4, la portion centrale permet de générer une mobilité en latéralité de l'articulation. La forme et l'épaisseur des volets permet alors d'ajuster les efforts pour permettre la flexion et également la plage de mouvement autorisée. Il suffit pour cela de faire varier la section de la bande utilisée pour les volets, la hauteur des volets et leur nombre et éventuellement, les butées.

A titre d'exemple, il a été réalisé des essais avec une partie centrale, l'une comportant cinq volets identiques, de section 0,17 mm, de hauteur 6,6 mm, de largeur 6,7 mm et de longueur 7mm. Avec cette géométrie, il a été mesuré la contrainte nécessaire à l'obtention de la déformation de la portion centrale dans une position à 90° en flexion, soit une charge de 120 g. La déformation interne est alors de 0,31 % dans ce cas précis.

De plus, l'axe de rotation de la partie centrale (1) est mobile au cours de la flexion, de la même façon que celui d'une articulation saine. La position de la zone de liaison entre les parties d'ancrage (2, 3) et la portion centrale (1) permet alors de modifier le déplacement de l'axe de rotation lors de la flexion.

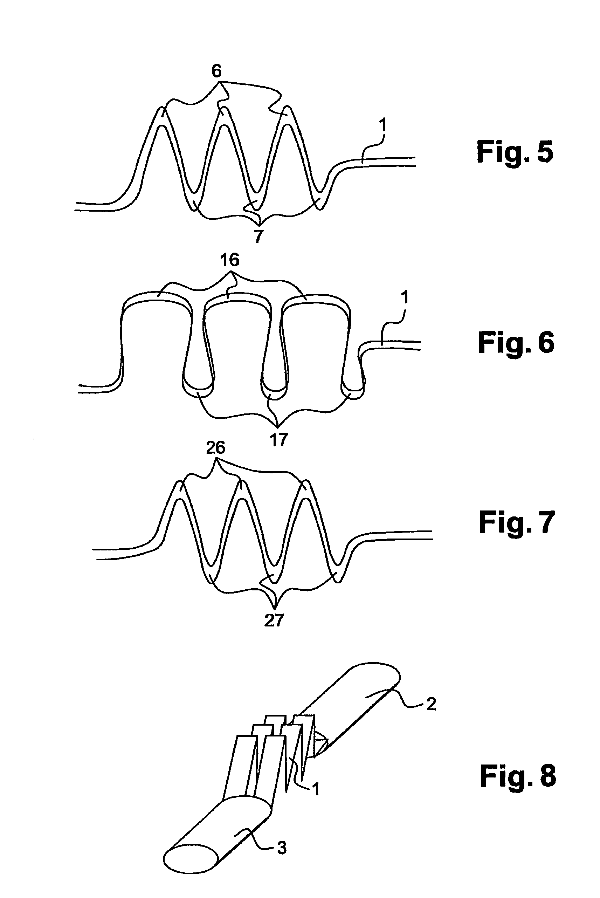

Tel que représenté à la figure 5, les plis (6) situés dans la zone supérieure de la portion centrale (1) sont sensiblement identiques aux plis (7) situés dans la partie inférieure de la portion (1).

Selon un autre mode de réalisation tel que représenté à la figure 6, les plis (16) situés dans la partie supérieure de la portion (1) sont différents des plis (17) situés dans la partie inférieure de la portion centrale (1).

Tel que représenté à la figure 7, les plis sont disposés de manière symétrique par rapport à un plan contenant les axes des parties d'ancrage (2,3). Ainsi, les plis (26) se situent à la même distance de ce plan que les plis (27).

Il est également possible de rendre asymétriques les plis par rapport au plan contenant les axes des parties d'ancrage. De cette manière, il est possible de faciliter la flexion ou l'extension en déplaçant la hauteur de l'axe de rotation. Ceci peut être envisagé lorsque des ligaments sont endommagés, car un déplacement dorsal de l'axe de rotation facilite la flexion et un déplacement palmaire facilite l'extension.

Tel que représenté à la figure 8, la portion centrale (1) comporte des volets ajourés. De cette manière, cela revient à disposer deux volets de très faible largeur, l'un à côté de l'autre. Il est bien entendu que l'invention ne se limite pas à cette unique variante et peut présenter une pluralité de volets disposés l'un par rapport à l'autre.

On observe également que la prothèse peut, dans une première variante, se présenter sous la forme d'un ensemble monobloc, les deux parties d'ancrage (2) et (3) et la portion centrale (1) étant solidaires.

Dans une seconde variante, il est possible de rapporter la portion centrale (1) entre les deux parties d'ancrage (2) et (3) de façon à permettre le changement ultérieur de cette portion, par exemple lorsque la mobilité de l'articulation n'est plus adaptée ou plus simplement lorsque la portion centrale a perdu de son élasticité.

Il ressort de ce qui précède que la prothèse conforme à l'invention présente de multiples avantages et notamment :
elle permet de fournir une liaison pivot dont l'axe de rotation est mobile à la manière d'une articulation saine ;
elle autorise une capacité de mouvement en latéralité ;
elle est réalisée en un matériau qui n'est pas dangereux pour l'organisme, et qui permet une grande longévité de la prothèse grâce à l'élasticité du matériau ;
elle permet une grande stabilité en compression lors de la flexion ;
les plis permettent de diminuer la déformation locale et permettent de régler les butées de latéralité.

Comme indiqué, l'invention trouve une application avantageuse dans le cadre de fractures interphalangiennes, métacarpophalangiennes, et métatarsophalangiennes.

## Revendications

1. Prothèse interphalangienne et/ou métacarpophalangienne comportant une portion centrale déformable (1) et deux parties d'ancrage (2) et (3) situées de part et d'autre de cette portion centrale (1), la portion centrale (1) comportant au moins deux volets articulés l'un par rapport à l'autre, en faisant office de plis aptes à permettre au moins un mouvement de flexion, **caractérisée en ce que** ladite prothèse est dans un alliage à mémoire de forme pour avoir une grande élasticité.

2. Prothèse selon la revendication 1, **caractérisée en ce que** l'alliage à mémoire de forme comporte du titane et du nickel.

3. Prothèse selon l'une quelconque des revendications 1 à 2, **caractérisée en ce que** chaque volet est apte à se déformer en torsion.

4. Prothèse selon l'une quelconque des revendications 1 à 3, **caractérisée en ce que** les volets de la portion centrale (1) sont ajourés longitudinalement

5. Prothèse selon l'une quelconque des revendications 1 à 4, **caractérisée en ce que** la section des volets est variable.

6. Prothèse selon l'une quelconque des revendications 1 à 5, **caractérisée en ce que** les plis sont symétriques entre eux.

7. Prothèse selon l'une quelconque des revendications 1 à 5, **caractérisée en ce que** les plis sont asymétriques entre eux.

8. Prothèse selon l'une quelconque des revendications 1 à 7, **caractérisée en ce que** les plis sont disposés symétriquement par rapport aux parties d'ancrage.

9. Prothèse selon l'une quelconque des revendications 1 à 7, **caractérisée en ce que** les plis sont disposés de manière décalée par rapport aux parties d'ancrage.

## Claims

1. Interphalangeal and/or metacarpophalangeal prosthesis comprising a central deformable portion (1) and two anchoring parts (2) and (3) located on either side of this central portion (1), the central portion (1) comprising at least two flaps hinged to one another, acting as folds suitable for allowing at least a bending movement, **characterised in that** the said prosthesis is made from a shape memory alloy so as to have a high elasticity.

2. Prosthesis according to Claim 1, **characterised in that** the shape memory alloy comprises titanium and nickel.

3. Prosthesis according to either of Claims 1 and 2, **characterised in that** each flap is suitable for being deformed by twisting.

4. Prosthesis according to any one of Claims 1 to 3, **characterised in that** the flaps of the central portion (1) are slotted longitudinally.

5. Prosthesis according to any one of Claims 1 to 4, **characterised in that** the flaps have a variable cross section.

6. Prosthesis according to any one of Claims 1 to 5, **characterised in that** the bends are symmetrical to one another.

7. Prosthesis according to any one of Claims 1 to 5, **characterised in that** the bends are asymmetrical to one another.

8. Prosthesis according to any one of Claims 1 to 7, **characterised in that** the bends are disposed symmetrically about the anchoring parts.

9. Prosthesis according to any one of Claims 1 to 7, **characterised in that** the bends are disposed in an offset manner with regard to the anchoring parts.

## Patentansprüche

1. Interphalangeale und / oder metacarpophalangeale Gelenkprothese mit einem verformbaren mittleren Teil (1) und zwei Verankerungsabschnitten (2) und (3), die zu beiden Seiten dieses mittleren Teils (1) angeordnet sind, wobei der mittlere Teil (1) mindestens zwei zueinander bewegliche Klappen besitzt, die als Falten dienen, so dass mindestens eine Biegebewegung möglich ist, **dadurch gekennzeichnet, dass** die Prothese aus einer Legierung mit Formgedächtnis besteht, so dass eine hohe Elastizität erreicht wird.

2. Prothese nach Anspruch 1, **dadurch gekennzeichnet, dass** die Legierung mit Formgedächtnis Titan und Nickel aufweist.

3. Prothese nach einem der Ansprüche 1 bis 2, **dadurch gekennzeichnet, dass** sich jede Klappe beim Drehen verformen kann.

4. Prothese nach einem der Ansprüche 1 bis 3, **dadurch gekennzeichnet, dass** die Klappen des mittleren Teils (1) längs durchbrochen sind.

5. Prothese nach einem der Ansprüche 1 bis 4, **dadurch gekennzeichnet, dass** der Querschnitt der Klappen variabel ist.

6. Prothese nach einem der Ansprüche 1 bis 5, **dadurch gekennzeichnet, dass** die Falten untereinander symmetrisch sind.

7. Prothese nach einem der Ansprüche 1 bis 5, **dadurch gekennzeichnet, dass** die Falten untereinander asymmetrisch sind.

8. Prothese nach einem der Ansprüche 1 bis 7, **dadurch gekennzeichnet, dass** die Falten gegenüber den Verankerungsabschnitten symmetrisch angeordnet sind.

9. Prothese nach einem der Ansprüche 1 bis 7, **dadurch gekennzeichnet, dass** die Falten gegenüber den Verankerungsabschnitten versetzt angeordnet sind.
